# EUROPEAN PATENT SPECIFICATION

(11) **EP 3 530 223 B1**
(45) Date of publication and mention of the grant of the patent: **22.05.2024**
(21) Application number: 16919581.5
(22) Date of filing: 20.10.2016
(51) Int. Cl.: A61B 17/58, A61B 17/56, A61B 17/72, A61B 17/80

(54) **TWO-WAY FIXING STEEL PLATE AND BONE FIXING SYSTEM**
ZWEIWEGE-FIXIERSTAHLPLATTE UND KNOCHENFIXIERUNGSSYSTEM
PLAQUE D'ANGLE POUR FIXATION BIDIRECTIONNELLE ET SYSTÈME DE FIXATION OSSEUSE ASSOCIÉ

(43) Date of publication of application: 28.08.2019
(73) Proprietor: Chen, Hua, Beijing 100853 (CN); Tang, Peifu, Beijing 100853 (CN); Tianjin Zhengtian Medical Instrument Co., Ltd., Airport Economic Zone Tianjin, 300308 (CN)
(72) Inventor: CHEN, Hua, Beijing 100853 (CN); TANG, Peifu, Beijing 100853 (CN)
(74) Representative: Grünecker Patent- und Rechtsanwälte PartG mbB
(86) International application number: PCT/CN2016/102738
(87) International publication number: WO 2018/072181

(56) References cited:
- EP-A1- 1 398 001
- WO-A1-2017/035302
- WO-A2-2009/042783
- WO-A2-2010/031098
- CN-A- 102 421 382
- CN-A- 102 949 228
- CN-A- 104 771 221
- US-A- 5 352 228
- US-A1- 2003 069 581
- US-A1- 2013 030 435
- US-A1- 2015 105 779

## Description

### Technical Field

The present invention relates to the technical field of medical devices, and in particular to a bidirectional fixation steel plate and a bone shaft fixation system used for bone nonunion or fracture non-healing after a bone shaft fracture.

### Background Technique

In the prior art, a bone fracture is divided into stable fracture and unstable fracture according to the degree of stability of the fracture end. For stable fracture, it usually was treated with manipulations and external fixation methods such as a plaster, a splint, a brace, or traction brake method, etc., are used to maintain stable fixation and achieve final healing; for unstable fracture, surgical treatment is required, which uses an internal fixation system and an external fixation system, and the internal fixation system is divided into a screw-plate fixation system and an intramedullary nail fixation system. However, regardless of which treatment method is adopted, large-sample follow-up studies have showed that some patients may suffer from fracture nonunion or delayed healing, with an incidence rate of up to 10%.

From US 2013/0030435 A1 a method of stabilizing a fractured bone is known that includes: a) providing a first support member in a medullary canal of a fractured bone; b) providing a second support member outside the fractured bone; c) providing a transverse connector adapted to link the first support member to the second support member in an angle-stable connection; d) connecting the transverse connector to the first and second support members in an angle-stable connection; e) providing one or more bone screws adapted to be connected to the second support member in an angle-stable connection; and f) implanting the bone screws such that the shank of the screw is implanted in the fractured bone while the head of the screw is connected to the second support member in an angle-stable connection. The system provides superior fixation of long bones that allows for early functional range of motion without loss of alignment or fixation.

Moreover, WO 2009/042783 A2 suggests a device for treating a bone that includes a plate having a connector opening and a first fastener receiving lumen extending therethrough, the first fastener receiving lumen being sized and shaped to receive therethrough a bone fastener and a nail sized and shaped for insertion in a medullary canal of a target bone, the nail including an elongate shaft and an opening extending through the shaft in combination with a connector sized and shaped so that, when the plate is positioned over a target portion of bone in a desired alignment with the nail, the connector may be passed into the target portion of bone via the connector opening and into the opening of the nail, the shape of the connector engaging the opening to form an angularly stable connection between the plate and the nail.

Additionally, WO 2010/031098 A2 describes an implant, in particular an intramedullary pin for treating a proximal fracture of the humerus, said pin having at least one distal and at least one proximal part, which can be moved in relation to one another in order to modify the length of the implant and which have co-operating guide surfaces. The proximal and the distal part have co-operating stops for limiting the axial relative movement, said proximal part and distal part being capable of relatively free movement within the delimitations defined by the stops and each being provided with at least one transverse bore for receiving and/or fastening fixing means. The implant is also provided with means for locking the relative movement of the two parts about the implant axis.

Furthermore, US 2015/0105779 A1 discloses a surgical aiming system comprising a one or more fixation plates, an aiming assembly, a one or more nails and a plurality of screws. Said aiming assembly comprising a channeling assembly and a plurality of channels. Said one or more fixation plates comprising a plurality of plate apertures capable of receiving a portion of said plurality of screws. Said plurality of channels in said aiming assembly are capable of aligning a portion of said plurality of screws with said plurality of plate apertures in a first fixation plate. Said plurality of channels receive and align said plurality of screws as they are secured to said first bone. Said first fixation plate being extramedullary attached to a portion of said surface of said first bone; and said plurality of channels of said aiming assembly being aligned to identify a one or more intramedullary locations within said first bone.

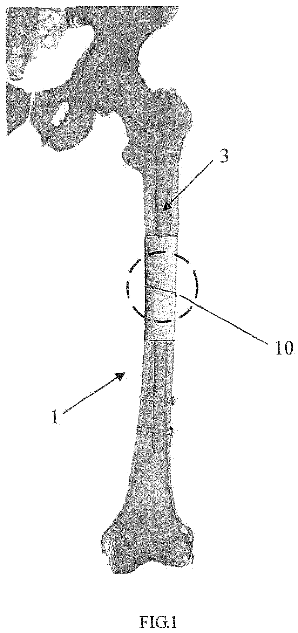

For patients suffering from fracture non-healing or delayed healing who are fixed by the intramedullary nail fixation system, besides the infection factors, these patients can be divided into two categories of multi-callus ones and few-callus ones according to the number of calluses formed at the fracture site; and can be divided into stable type and unstable type according to the degree of stability of fracture end. For patients with stable fracture end and multiple calluses, most of them can be treated by observation; while for patients with stable fracture end and few calluses, bone grafting treatment can be adopted. But for patients with unstable fracture, regardless of multiple calluses or few calluses, it is necessary to take various ways to strengthen the stability of the fracture end to promote the gradual healing of the fracture because of local instability of fracture. However, relying on the existing clinically available internal fixation methods, it is still impossible to perfectly solve the internal fixation problems such as defects of the fracture end, or unstable fracture end, non-healing or nonunion after comminuted fracture by adopting the intramedullary nail system for fixation (for example, nonunion 10 of a bone shaft 1 after fracture as shown in dotted round frame in Fig.1), that is, there is no stable medical device designed specifically for nonunion and fracture non-healing. Therefore, it is urgent to develop an internal fixation apparatus, a system or a method for nonunion and non-healing after fracture.

### Summary of the invention

To solve the problems of unstable fracture end, fracture non-healing and delayed healing occurring in the prior art after the bone shaft fracture site is fixed using an intramedullary nail system, the present invention proposes a bidirectional fixation steel plate. By using a steel plate body with a structure matched with a front side of the bone shaft, by providing several guide holes for locking screws to pass through in the steel plate body, and further through matching of the guide holes with first locking screws to control the rotation and axial stability of the intramedullary nail, the bidirectional fixation steel plate may achieve stable fixation of the fracture end, prevent dislocation of the fracture end, and guarantee to assist in accurate positioning, reduction and union of fracture site. The present invention also relates to a bone shaft fixation system.

The technical solution of the present invention is as follows:
A bidirectional fixation steel plate, comprises a steel plate body, wherein the steel plate body is used to be implanted from a front side of a bone shaft and has a structure matched with the front side of the bone shaft to fixedly fit with a fracture end, the steel plate body is provided with at least two pairs of guide holes for first locking screws to pass through respectively in the structure matched with the front side of the bone shaft, and angles of the guide holes enable all the first locking screws passing through the guide holes to clamp an intramedullary nail together to control rotation and axial stability of the intramedullary nail.

Positioning holes for second locking screws to pass through are provided at a distal end and/or a proximal end of the steel plate body, and angles of the positioning holes enable the second locking screws passing through the positioning holes to position the steel plate body in the position in close contact with the bone shaft.

The steel plate body is manufactured by an integral molding process.

The steel plate body has a long axis consistent with the intramedullary nail when implanted, and respective pairs of guide holes are arranged along the long axis of the steel plate body, the guide holes being arranged in double columns.

The steel plate body is provided with two pairs of guide holes in the structure matched with the front side of the bone shaft, each pair of guide holes are arranged along a short axis of the steel plate body, and the guide holes are divided into two rows along the long axis of the steel plate body.

The steel plate body is provided with four pairs of guide holes in the structure matched with the front side of the bone shaft, each pair of guide holes are arranged along the short axis of the steel plate body, and the guide holes are divided into four rows along the long axis of the steel plate body.

A bone shaft fixation system, comprises the foregoing bidirectional fixation steel plate, and further comprises an intramedullary nail and several first locking screws, wherein the intramedullary nail is used to fix a fracture end and has a structure matched with an intramedullary cavity of bone shaft, and each of the first locking screws passes through corresponding guide hole respectively and then is closely fitted with a side surface of the intramedullary nail and fixedly clamp the intramedullary nail together for controlling the rotation and axial stability of the intramedullary nail.

The bone shaft fixation system further comprises second locking screws passing through the positioning holes for positioning the bidirectional fixation steel plate.

Each of the first locking screws and the second locking screws has a diameter ranging from 2.4 to 4.5 mm.

The first locking screws and/or the second locking screws are hollow locking screws or solid locking screws.

The technical effects of the present invention are as follows:
The present invention relates to a bidirectional fixation steel plate, comprising the steel plate body for implantation from a front side of the bone shaft and has a structure matched with the front side of the bone shaft to fixedly fit with the fracture end, the steel plate body is provided with at least two pairs of guide holes for the first locking screws to pass through, and preferably, the steel plate body is integrally molded, each pair of guide holes having respective specific angles to respectively match with the first locking screws during application to achieve specific functions. According to the bidirectional fixation steel plate proposed by the present invention, the steel plate body with a structure matched with the front side of the bone shaft is used to be implanted from the front side of the bone shaft and fix the bone shaft fracture end, and the steel plate body may also support the bone shaft fracture end to enhance the stability of the fracture end and effectively guarantee to assist in stable reduction and healing of the fracture site; the steel plate body with a specific structure is matched with the guide holes arranged in pairs and provided with specific angles, in this way, after passing through in a direction of the guide holes, the first locking screws may clamp the intramedullary nail together to control the rotation and axial stability of the intramedullary nail. In addition, the guide holes may also be considered as universal locking holes, which may achieve that the steel plate body is in close contact with the bone shaft (that is, the bidirectional fixation steel plate is closely fitted with the bone shaft) and achieve that the bone shaft fracture end is supported and the steel plate body is locked. Thus, the unique structure of the present invention may achieve anti-rotation and anti-axial instability of the bone shaft fracture end, control the rotation of the intramedullary nail and strengthen support, thus improving the stability of the fracture end; moreover, during actual applications, the steel plate body may be minimally invasively implanted from the front side of the bone shaft and the implantation of the first locking screws can be prevented from being interfered with the original fixation. The bidirectional fixation steel plate is simple in structure and compact in size, may be implanted through the incision at the front side of the bone shaft, and through the specific angles at which the guide holes are arranged, may be made to achieve support, anti-rotation and axial stability of the bone shaft fracture end when matched with the first locking screws, to further enhance the reliability of fixation after reduction, thereby being beneficial to fracture healing.

For the bidirectional fixation steel plate proposed by the present invention, positioning holes for the second locking screws to pass through are provided at the distal end and/or proximal end of the steel plate body, in this way, after passing through in a direction of the positioning holes, the second locking screws may position the steel plate body in a position in close contact with the bone shaft, to further improve the accuracy of fixation after reduction, enhancing the fracture healing effect.

In addition, the guide holes arranged in pair proposed by the present invention may be sequentially arranged along the long axis of the steel plate body according to the pair number thereof. All the guide holes are arranged in multiple rows and double columns, and the specific arrangement structure may be reasonably and selectively set according to the specific conditions of the fracture site. For example, the guide holes may be divided into two rows, four rows, six rows, eight rows, etc., along the long axis of the steel plate body, in order to match with the fracture end to achieve optimal effects of anti-rotation and anti-axial instability while supporting and stabilizing the fracture end, so as to accelerate and promote fracture healing and make the fracture healing state better.

The present invention further relates to a bone shaft fixation system comprising the above bidirectional fixation steel plate and the first locking screws, and further comprising an intramedullary nail, wherein the above components are cooperatively used during actual applications, the long axis of the steel plate body of the bidirectional fixation steel plate is consistent with the intramedullary nail, the intramedullary nail achieves effective support and fixation of the fracture end, each of the first locking screws passing through the bidirectional fixation steel plate is closely fitted with the intramedullary nail and is fixed through the cortex to achieve accurate positioning and fixation of the bone shaft fracture site and anti-rotation and anti-axial instability, so that the steel plate body achieves the effects of controlling rotation and strengthening support of the intramedullary nail by bidirectional fixation screws (or bidirectional locking screws) through cooperation of the guide holes provided with specific angles and respective first locking screws, further strengthens the effective support and stability for the bone shaft fracture, assists in the accurate positioning and fixation of the fracture site, and effectively ensures the stability after reduction of the bone shaft fracture.

### Description of the Drawings

Fig. 1 is a schematic diagram of bone nonunion after bone shaft fracture.
Fig. 2 is a front view of one preferred structure of a bidirectional fixation steel plate of the present invention.
Fig. 3 is a structural side view showing the bidirectional fixation steel plate of Fig. 2 in a used state.
Fig. 4 is a structural perspective view showing the bidirectional fixation steel plate of Fig. 2 in a used state.
Fig. 5 is a top view of Fig. 4.
Fig. 6 is a front view of another preferred structure of a bidirectional fixation steel plate of the present invention.
Fig. 7 is a structural side view showing a preferred bone shaft fixation system of the present invention in a used state.
Fig. 8 is a structural perspective view showing the bone shaft fixation system of Fig. 7 in a used state.

Reference numbers in the drawings are listed as follows:
1-bone shaft; 10-nonunion; 2-bidirectional fixation steel plate; 20-steel plate body; 2101-guide hole; 2102-guide hole; 2103-guide hole; 2104-guide hole; 2105-guide hole; 2106-guide hole; 2107-guide hole; 2108-guide hole; 2201-first locking screw; 2202-first locking screw; 2203-first locking screw; 2204-first locking screw; 2205-first locking screw; 2206-first locking screw; 2207-first locking screw; 2208-first locking screw; 23-positioning hole; 3-intramedullary screw.

### Detailed Description of the preferred Embodiments

The present invention will be further described with reference to the accompanying drawings.

The present invention relates to a bidirectional fixation steel plate, comprising a steel plate body, wherein the steel plate body is used to be implanted from a front side of the bone shaft and has a structure matched with the front side of the bone shaft to fixedly fit with the fracture end, the steel plate body is provided with at least two pairs of guide holes for the first locking screws to pass through respectively in the structure matched with the front side of the bone shaft, and the angles of the guide holes enable the first locking screws passing through to clamp the intramedullary nail together to control the rotation and axial stability of the intramedullary nail. The specific structural shape of the steel plate body, and the specific positions of each pair of guide holes and the number of the guides holes may be reasonably set according to actual application situations, are not limited by the present invention and include but are not limited to the above range. For example, the steel plate body may be designed into a rectangular structure and an inner side thereof may be closely fitted with the front side of the bone shaft, or may be designed into other reasonable structures. In addition, the steel plate body has a long axis consistent with the intramedullary nail when implanted, and each pair of guide holes are arranged along the long axis of the steel plate body, the guide holes being arranged in double columns, that is, the guide holes may be arranged in multiple rows and double columns finally. For example, the steel plate body is provided with two pairs of guide holes in the structure matched with the front side of the bone shaft, each pair of guide holes are arranged along a short axis of the steel plate body, and the guide holes are divided into two rows along the long axis of the steel plate body. Alternatively, the steel plate body is provided with four pairs of guide holes in the structure matched with the front side of the bone shaft, each pair of guide holes are arranged along the short axis of the steel plate body, and are divided into four rows along the long axis of the steel plate body. The specific arrangement structure may be reasonably and selectively set according to the specific conditions of the fracture site. For example, the guide holes may be divided into two rows, four rows, six rows, eight rows, etc., along the long axis of the steel plate body, in order to match with the fracture end to achieve optimal anti-rotation and anti-axial instability while supporting and stabilizing the fracture end, so as to accelerate and promote fracture healing and make the fracture healing state better. In other words, for the bidirectional fixation steel plate of the present invention, the length of the bidirectional fixation steel plate may be determined according to the number of the guide holes provided in the structure of the steel plate body matched with the front side of the bone shaft. For example, the length of the bidirectional fixation steel plate may be determined according to two rows, four rows, six rows, eight rows or more rows of guide holes.

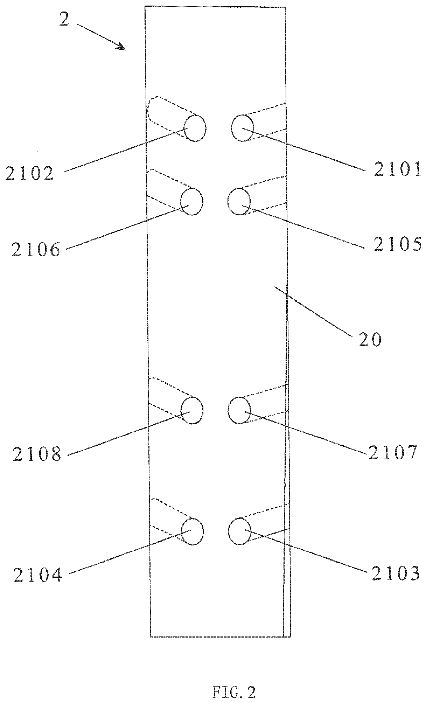

Fig. 2 is a front view of one preferred structure of a bidirectional fixation steel plate 2 of the present invention. As shown in Fig. 2, the fixation steel plate 2 comprises a steel plate body 20 which is preferably made of stainless steel by an integral molding process. The steel plate body 20 is used to be implanted from a front side of the bone shaft and has a structure matched with the front side of the bone shaft to fixedly fit with the fracture end, support and stabilize the fracture end, and promote fracture healing. The steel plate body 20 of the embodiment is preferably provided with four pairs of guide holes (for example, reference numerals 2101-2108, i.e. guide hole 2101 and guide hole 2102, guide hole 2103 and guide hole 2104, guide hole 2105 and guide hole 2106, guide hole 2107 and guide hole 2108) for the first locking screws to pass through in the structure matched with the front side of the bone shaft. Each of the guide holes is provided with a specific angle, when performing locking and fixing using the first locking screws passing through along the angles of the guide holes, the specific angles directly enable the steel plate body 20 to be in close contact with the bone shaft and enable the first locking screws passing through to clamp the intramedullary nail together to control the rotation and axial stability of the intramedullary nail, that is to say, achieve a close fit between the steel plate body 20 and the front side of the bone shaft as well as fixation and anti-rotation of the intramedullary nail, and then achieve axial stability, fixed support and anti-rotation for the bone shaft fracture end. In addition, as shown in Fig. 2, the guide hole 2101, the guide hole 2105, the guide hole 2107 and the guide hole 2103 are arranged in an approximate straight line, and the guide hole 2102, the guide hole 2106, the guide hole 2108 and the guide hole 2104 are arranged in an approximate straight line, that is, all the guide holes are arranged in four rows and double columns, and the arrangement shape may also be set in any other mode as long as it ensures the effects of effective fixation, anti-rotation, anti-axial instability and ensures minimum injury to patients during actual applications. The bidirectional fixation steel plate 2 proposed by the present invention adopts an integrally molded steel plate body 20 which is implanted from the front side of the bone shaft and has a structure matched with the front side of the bone shaft, and may achieve the good effect of fitting and fixing the fracture end. Meanwhile, the steel plate body 20 may be matched with the first locking screws to achieve the axial positioning and fixation of the long bone shaft fracture area and prevent the dislocation of the fracture site, and may stabilize and support the fracture non-healing or nonunion site at the fracture end, which effectively ensures the stability fracture healing, further guarantees to assist in the accurate positioning, reduction and healing of the fracture site, accelerates and promotes fracture healing and makes the fracture healing state better.

The working principle and use method of the bidirectional fixation steel plate 2 proposed by the present invention are specifically described as follows:

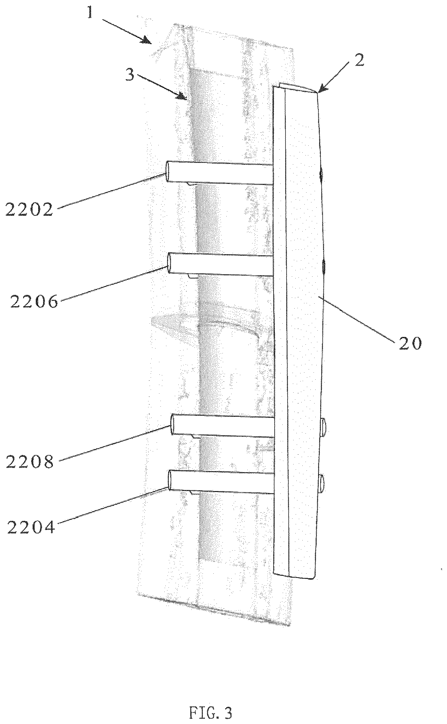

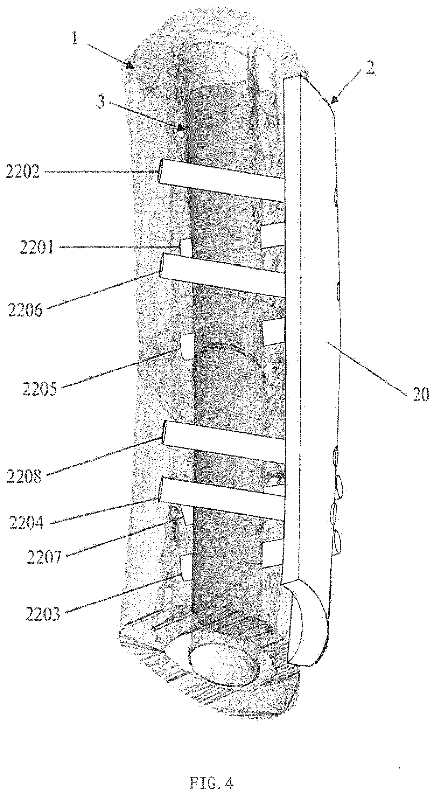

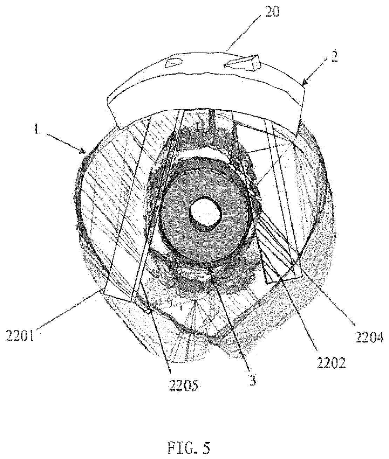

Figs. 3 to 5 are structural schematic diagrams showing a preferred bidirectional fixation steel plate 2 proposed by the present invention in a used state. During actual applications, first, at the fracture site, for example, the front side of the bone shaft 1, that is, the front of the nonunion site, skin, subcutaneous tissue, quadriceps muscle abdomen and periosteum are cut along a long axis of the limb to expose the nonunion site, and then a bone cortex cutting operation is performed at the nonunion site using a sharp osteotome, to fully expose the nonunion site; bone paste operation and cortex bone jump are respectively performed using a minimally invasive bone extraction drill; bone paste is filled in the nonunion site, cancellous bone strip is paved under the cut bone cortex; stripping under periosteum is made using the extensibility of skin and muscle; then, the bidirectional fixation steel plate 2 shown in Fig. 2 is implanted under the periosteum, the steel plate body 20 is implanted at the front side of the bone shaft, and the inner side of the steel plate body 20 is closely fitted with the front side of the bone shaft, to achieve good effects of support, anti-rotation and fixation, perfectly achieving effective support and fixation for bone shaft fracture, assisting in accurate positioning and healing of the fracture site, and effectively ensuring the stability of the reduction and healing of the bone shaft fracture. As shown in Fig. 3 and Fig. 4 (wherein Fig. 3 is side view, and Fig. 4 is a perspective view), the first locking screw 2201 and the first locking screw 2202 respectively pass through the guide hole 2101 and the guide hole 2102 provided in the steel plate body 20 at a certain angle, the first locking screw 2205 and the first locking screw 2206 respectively pass through the guide hole 2105 and the guide hole 2106 provided in the steel plate body 20 at a certain angle, the first locking screw 2207 and the first locking screw 2208 respectively pass through the guide hole 2107 and the guide hole 2108 provided in the steel plate body 20 at a certain angle, the first locking screw 2203 and the first locking screw 2204 respectively pass through the guide hole 2103 and the guide hole 2104 provided in the steel plate body 20 at a certain angle, to closely fit the steel plate body 20 with the front side of the bone shaft so as to fix the fracture end, and the steel plate body 20 cooperates with respective guide holes to make all locking screw passing through the guide holes to clamp the intramedullary nail 3 together to control the rotation and axial stability of the intramedullary nail 3, thereby achieving axial support, fixation, anti-rotation and axial stability of the intramedullary nail 3. Moreover, each of the above certain angles may be reasonably set according to the actual situation as long as it ensures that each pair of first locking screws may effectively clamp the intramedullary nail 3, that is, achieves the effects of effective fixation, anti-rotation, anti-axial instability and ensure minimum injury to patients during actual applications. In addition, as shown in Fig. 5 (Fig. 5 is a top view of Fig. 4, i.e. a view from the proximal to the distal end of the bone shaft), the above four pairs of first locking screws, eight in total, are arranged in two approximate straight lines, that is, arranged in four rows and double columns, and each of the first locking screws passing through the steel plate body 20 is closely fitted with the intramedullary nail 3 and is fixed through the cortex (i.e. between the cortex and the periosteum), to clamp the intramedullary nail 3 together so as to control the rotation and axial stability of the intramedullary nail 3, and strengthen the effects of axial support, anti-rotation and stability of the intramedullary nail 3. After fixation and support by the bidirectional fixation steel plate 2 of the present invention, a negative pressure drainage tube is intubated, and periosteum, myofascial fascia, deep fascia, subcutaneous tissue and skin are closed by suture; and the drainage tube is pulled out 48 hours after the operation. Then, the patient with fracture takes daily exercises of passive extreme knee motion, lower limb isometric contraction, and weight-bearing activities under pain tolerance (toes on ground by 5 kg). The patient receives X-ray examination monthly to determine the weight-bearing process according to fracture healing condition until the patient fully recovers.

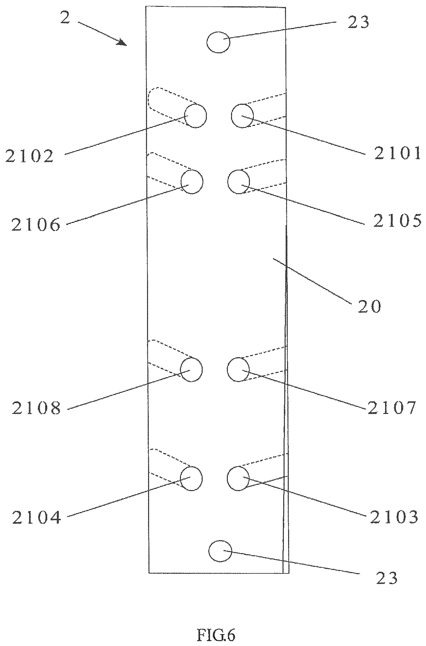

Fig. 6 is a front view of another preferred structure of a bidirectional fixation steel plate of the present invention. In this embodiment, positioning holes 23 for the second locking screw to pass through are provided at both the distal end and proximal end of the steel plate body 20 (that is to say, at an upper end portion and a lower end portion of the steel plate body), and the angles of the positioning holes enable the second locking screws passing through the positioning holes to position the steel plate body 20 in a position in close contact with the bone shaft, thus further improving the accurate positioning and fixation of the bone shaft fracture end, and enhancing the fracture healing effect.

The present invention further relates to a bone shaft fixation system, comprising the above bidirectional fixation steel plate, and further comprises an intramedullary nail and several first locking screw, wherein the bidirectional fixation steel plate may adopt the bidirectional fixation steel plate as shown in Figs. 1 to 5, the intramedullary nail is used to fix the fracture end and has a structure matched with an intramedullary cavity of the bone shaft, and each of the first locking screws passes through corresponding guide hole respectively and then is closely fitted with the side surface of the intramedullary nail and fixedly clamp the intramedullary nail together for controlling the rotation and axial stability of the intramedullary nail.

Preferably, the bone shaft fixation system may further comprise second locking screws passing through the positioning holes for positioning the bidirectional fixation steel plate. In this case, the bidirectional fixation steel plate adopts the structure as shown in Fig. 6, that is, positioning holes for the second locking screws to pass through may also be provided at the distal end and/or proximal end of the steel plate body (that is, the upper end portion and/or the lower end portion of the steel plate body), and the angles of the positioning holes enable the second locking screws passing through to position the steel plate body in a position in close contact with the bone shaft, so as to achieve accurate positioning and fixation of the bone shaft fracture end, and enhancing the fracture healing effect.

Preferably, the locking screws (the first locking screws and the second locking screws) for fixing the bidirectional fixation steel plate may have a diameter ranging from 2.4 mm to 4.5 mm; and the locking screws for fixing the bidirectional fixation steel plate (the first locking screws and the second locking screws) may be hollow locking screws or solid locking screws.

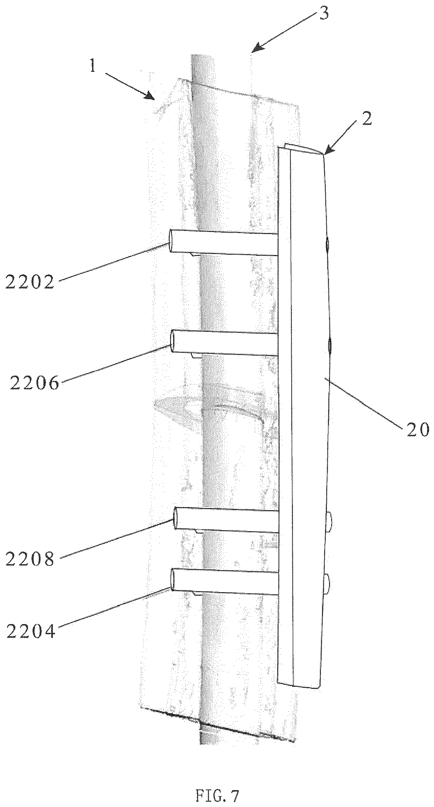

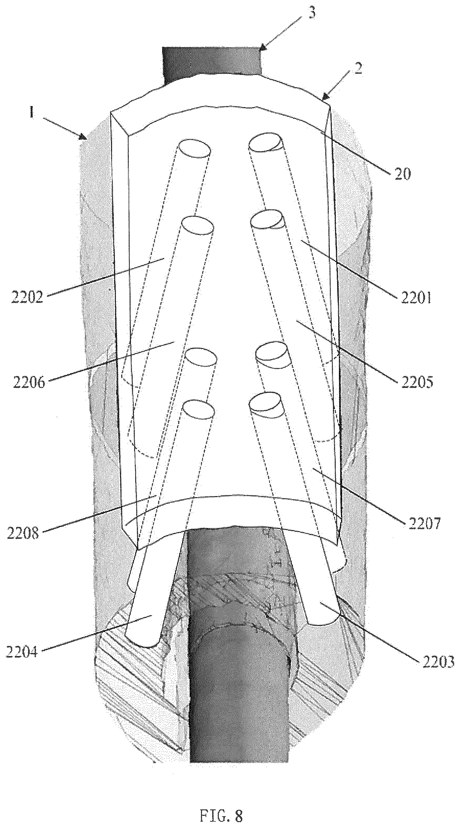

Fig. 7 and Fig. 8 are structural schematic diagrams showing a preferred bone shaft fixation system of the present invention in a used state. Fig. 7 is side view, and Fig. 8 is a perspective view, in which the bidirectional fixation steel plate 2 and the intramedullary nail 3 shown in Fig. 3 or Fig. 4 are included. Fixation of the bidirectional fixation steel plate 2, and clamping and fixation of the intramedullary nail 3 are conducted by a plurality of first locking screws as shown in Figs. 3 to 4 in the above-mentioned mode, to control rotation and strengthen the support. The intramedullary nail 3 has a structure matched with an intramedullary cavity of the bone shaft, first locking screws respectively passing through respective pairs of guide holes are closely fitted with the side surface of the intramedullary nail and control the rotation and axial stability of the intramedullary nail 3, that is to say, all the first locking screws passing through the steel plate body 20 are closely fitted with the intramedullary nail 3 and are fixed through the cortex (that is, between the cortex and the periosteum), and clamp the intramedullary nail 3 together to control the rotation and axial stability of the intramedullary nail 3, thus an overall structure that all the first locking screws tangentially embrace the intramedullary nail 3 is formed, to further strengthen the effects of axial support, anti-rotation and stability of the intramedullary nail 3, and then achieve support, anti-rotation and axial stability of the bone shaft fracture end.

The working principle and use method of the bone shaft fixation system proposed by the present invention are specifically described as follows:
As shown in Fig. 7 and Fig. 8, for the bone shaft fixation system proposed by the present invention, in the actual use, the intramedullary nail 3 may be inserted into the intramedullary cavity of the bone shaft through the bone shaft fracture end, and the bidirectional fixation steel plate 2 may be implanted through a conventional anterolateral incision. Thus, only one incision is required to achieve the implantation of the bone shaft fixation system proposed by the present invention, the intraoperative injury surface, degree of injury and amount of bleeding are usually smaller, and postoperative healing and recovery speed are faster. The working principle and use method of the bidirectional fixation steel plate 2 shown in Figs. 2 to 5 are the same as those described above. The bidirectional fixation steel plate 2 and the intramedullary nail 3 cooperate with each other, the long axis of the steel plate body 20 of the bidirectional fixation steel plate 2 is consistent with the intramedullary nail 3, the intramedullary nail 3 achieves the effective support and fixation of the fracture end, each of the first locking screws passing through the bidirectional fixation steel plate 2 is closely fitted with the intramedullary nail 3 and is fixed through the cortex, that is, the steel plate body 20 achieves the effects of controlling rotation and strengthening support of the intramedullary nail 3 by the bidirectional fixing screws (or the bidirectional locking screws) through cooperation of respective guide holes provided with specific angles and respective first locking screws, and then achieves accurate positioning and fixation of the bone shaft fracture site as well as anti-rotation and anti-axial instability, further strengthens the effective support and stability for the bone shaft fracture, and assists in the accurate positioning and fixation of the fracture site, thereby effectively ensuring the stability after reduction of the bone shaft fracture.

It should be noted that the foregoing described specific embodiments may enable those skilled in the art to more fully understand the present invention rather than limit the present invention in any way. Therefore, although the present invention has been described in detail with reference to the drawings and embodiments, those skilled in the art should understand that modifications or equivalent replacements can be made to the present invention.

## Claims

1. A bone shaft fixation system, comprising a bidirectional fixation steel plate (2) and an intramedullary nail (3), the bidirectional fixation steel plate comprising a steel plate body (20), wherein the steel plate body is configured to be used to be implanted from a front side of a bone shaft (1) and has a structure configured to be matched with the front side of the bone shaft to fixedly fit with a fracture end, the steel plate body is provided with at least two pairs of guide holes (2101-2108) for first locking screws (2201-2208) to pass through respectively in the structure configured to be matched with the front side of the bone shaft (1), and the guide holes (2101-2108) are provided with specific angles which enable all the first locking screws passing through the guide holes to clamp the intramedullary nail together to control rotation and axial stability of the intramedullary nail (3).

2. The bone shaft fixation system according to claim 1, wherein positioning holes (23) for second locking screws to pass through are provided at a distal end and/or a proximal end of the steel plate body (20), and angles of the positioning holes (23) enable the second locking screws passing through the positioning holes to position the steel plate body (20) in the position in close contact with the bone shaft (1).

3. The bone shaft fixation system according to claim 2, wherein the steel plate body (20) is manufactured by an integral molding process.

4. The bone shaft fixation system according to any of claims 1-3, wherein the steel plate body (20) has a long axis consistent with the intramedullary nail (3) when implanted, and respective pairs of guide holes are arranged along the long axis of the steel plate body, the guide holes being arranged in double columns.

5. The bone shaft fixation system according to claim 4, wherein the steel plate body is provided with two pairs of guide holes (2101-2108) in the structure matched with the front side of the bone shaft (1), each pair of guide holes (2101-2108) are arranged along a short axis of the steel plate body (20), and the guide holes (2101-2108) are divided into two rows along the long axis of the steel plate body (20).

6. The bone shaft fixation system according to claim 4, wherein the steel plate body (20) is provided with four pairs of guide holes (2101-2108) in the structure configured to be matched with the front side of the bone shaft (1), each pair of guide holes (2101-2108) are arranged along the short axis of the steel plate body (20), and the guide holes (2101-2108) are divided into four rows along the long axis of the steel plate body (20).

7. The bone shaft fixation system according to any of claims 1 to 6, wherein the bone shaft fixation system further comprising second locking screws passing through the positioning holes (23) for positioning the bidirectional fixation steel plate (2).

8. The bone shaft fixation system according to any of claims 1 to 7, wherein each of the first locking screws (2201-2208) and the second locking screws has a diameter ranging from 2.4 to 4.5 mm.

9. The bone shaft fixation system according to any of claims 1 to 7, wherein the first locking screws (2201-2208) and/or the second locking screws are hollow locking screws or solid locking screws.

## Patentansprüche

1. Knochenschaft-Fixiersystem, das eine bidirektionale Fixierstahlplatte (2) und einen intramedullären Nagel (3) umfasst, wobei die bidirektionale Fixierstahlplatte einen Stahlplattenkörper (20) umfasst, wobei der Stahlplattenkörper dazu eingerichtet ist verwendet zu werden, um von einer Vorderseite eines Knochenschafts (1) implantiert zu werden, und eine Struktur aufweist, die dazu eingerichtet ist, an die Vorderseite des Knochenschafts angepasst zu werden, um fest zu einem Frakturende zu passen, der Stahlplattenkörper mit wenigstens zwei Paaren von Führungslöchern (2101-2108) für erste Verriegelungsschrauben (2201-2208) versehen ist, die jeweils in der Struktur, die dazu eingerichtet ist, an die Vorderseite des Knochenschafts (1) angepasst zu werden, hindurchgeführt werden, und die Führungslöcher (2101-2108) mit spezifischen Winkeln versehen sind, die es ermöglichen, dass alle ersten Verriegelungsschrauben, die durch die Führungslöcher hindurchgeführt werden, den intramedullären Nagel zusammenklemmen, um die Rotation und axiale Stabilität des intramedullären Nagels (3) zu steuern.

2. Knochenschaft-Fixiersystem nach Anspruch 1, bei dem an einem distalen Ende und/oder einem proximalen Ende des Stahlplattenkörpers (20) Positionierlöcher (23) für den Durchtritt von zweiten Verriegelungsschrauben vorgesehen sind und Winkel der Positionierlöcher (23) es den durch die Positionierlöcher hindurchtretenden zweiten Verriegelungsschrauben ermöglichen, den Stahlplattenkörper (20) in der Position in engem Kontakt mit dem Knochenschaft (1) zu positionieren.

3. Knochenschaft-Fixiersystem nach Anspruch 2, bei dem der Stahlplattenkörper (20) durch ein integrales Formgebungsverfahren hergestellt wird.

4. Knochenschaft-Fixiersystem nach einem der Ansprüche 1 bis 3, bei dem der Stahlplattenkörper (20) eine Längsachse aufweist, die mit dem implantierten intramedullären Nagel (3) übereinstimmt, und jeweilige Paare von Führungslöchern entlang der Längsachse des Stahlplattenkörpers angeordnet sind, wobei die Führungslöcher in Doppelspalten angeordnet sind.

5. Knochenschaft-Fixiersystem nach Anspruch 4, bei dem der Stahlplattenkörper mit zwei Paaren von Führungslöchern (2101-2108) in der an die Vorderseite des Knochenschafts (1) angepassten Struktur versehen ist, jedes Paar von Führungslöchern (2101-2108) entlang einer kurzen Achse des Stahlplattenkörpers (20) angeordnet ist und die Führungslöcher (2101-2108) in zwei Reihen entlang der langen Achse des Stahlplattenkörpers (20) unterteilt sind.

6. Knochenschaft-Fixiersystem nach Anspruch 4, bei dem der Stahlplattenkörper (20) mit vier Paaren von Führungslöchern (2101-2108) in der Struktur versehen ist, die dazu eingerichtet ist, an die Vorderseite des Knochenschafts (1) angepasst zu werden, wobei jedes Paar von Führungslöchern (2101-2108) entlang der kurzen Achse des Stahlplattenkörpers (20) angeordnet ist und die Führungslöcher (2101-2108) in vier Reihen entlang der langen Achse des Stahlplattenkörpers (20) unterteilt sind.

7. Knochenschaft-Fixiersystem nach einem der Ansprüche 1 bis 6, wobei das Knochenschaft-Fixiersystem weiterhin zweite Verriegelungsschrauben umfasst, die durch die Positionierlöcher (23) zur Positionierung der bidirektionalen Fixierstahlplatte (2) verlaufen.

8. Knochenschaft-Fixiersystem nach einem der Ansprüche 1 bis 7, bei dem jede der ersten Verriegelungsschrauben (2201-2208) und der zweiten Verriegelungsschrauben einen Durchmesser im Bereich von 2,4 bis 4,5 mm aufweist.

9. Knochenschaft-Fixiersystem nach einem der Ansprüche 1 bis 7, bei dem die ersten Verriegelungsschrauben (2201-2208) und/oder die zweiten Verriegelungsschrauben hohle Verriegelungsschrauben oder massive Verriegelungsschrauben sind.

## Revendications

1. Système de fixation de tige osseuse, comprenant une plaque d'angle de fixation bidirectionnelle (2) et un clou intramédullaire (3), la plaque d'angle de fixation bidirectionnelle comprenant un corps de plaque d'angle (20), dans lequel le corps de plaque d'angle est configuré pour être utilisé pour être implanté à partir d'un côté avant d'une tige osseuse (1) et présente une structure configurée, au côté avant de la tige osseuse, pour s'adapter de manière fixe à une extrémité de fracture, le corps de la plaque d'angle est pourvu d'au moins deux paires de trous de guidage (2101-2108) pour que les premières vis de verrouillage (2201-2208) passent respectivement dans la structure configurée pour s'adapter au côté avant de la tige osseuse (1), et
les trous de guidage (2101-2108) sont pourvus d'angles spécifiques qui permettent à toutes les premières vis de verrouillage passant par les trous de guidage de serrer le clou intramédullaire ensemble pour contrôler la rotation et la stabilité axiale du clou intramédullaire (3).

2. Système de fixation de la tige osseuse selon la revendication 1, dans lequel des trous de positionnement (23) pour le passage des secondes vis de verrouillage sont prévus à une extrémité distale et/ou une extrémité proximale du corps de plaque d'angle (20), et les angles des trous de positionnement (23) permettent aux secondes vis de verrouillage passant par les trous de positionnement de placer le corps de plaque d'angle (20) dans la position en contact étroit avec la tige osseuse (1).

3. Système de fixation de la tige osseuse selon la revendication 2, dans lequel le corps de plaque d'angle (20) est fabriqué par un procédé de moulage intégral.

4. Système de fixation de la tige osseuse selon l'une des revendications 1 à 3, dans lequel le corps de plaque d'angle (20) a un axe long cohérent avec le clou intramédullaire (3) lorsqu'il est implanté, et des paires respectives de trous de guidage sont disposées le long de l'axe long du corps de plaque d'angle, les trous de guidage étant disposés en doubles colonnes.

5. Système de fixation de la tige osseuse selon la revendication 4, dans lequel le corps de plaque d'angle est pourvu de deux paires de trous de guidage (2101-2108) dans la structure correspondant au côté avant de la tige osseuse (1), chaque paire de trous de guidage (2101-2108) est disposée le long d'un axe court du corps de plaque d'angle (20), et les trous de guidage (2101-2108) sont divisés en deux rangées le long de l'axe long du corps de plaque d'angle (20).

6. Système de fixation de la tige osseuse selon la revendication 4, dans lequel le corps de plaque d'angle (20) est pourvu de quatre paires de trous de guidage (2101-2108) dans la structure configurée pour s'adapter au côté avant de la tige osseuse (1), chaque paire de trous de guidage (2101-2108) sont disposés le long de l'axe court du corps de plaque d'angle (20), et les trous de guidage (2101-2108) sont divisés en quatre rangées le long de l'axe long du corps de plaque d'angle (20).

7. Système de fixation de la tige osseuse selon l'une des revendications 1 à 6, dans lequel le système de fixation de la tige osseuse comprend en outre des secondes vis de verrouillage passant à travers les trous de positionnement (23) pour positionner la plaque d'angle de fixation bidirectionnelle (2).

8. Système de fixation de la tige osseuse selon l'une des revendications 1 à 7, dans lequel chacune des premières vis de verrouillage (2201-2208) et des secondes vis de verrouillage a un diamètre compris entre 2,4 et 4,5 mm.

9. Système de fixation de la tige osseuse selon l'une quelconque des revendications 1 à 7, dans lequel les premières vis de verrouillage (2201-2208) et/ou les secondes vis de verrouillage sont des vis de verrouillage creuses ou des vis de verrouillage pleines.
